Europäisches Patentamt

European Patent Office

Office européen des brevets

(11) Publication number: **0 064 044**
**B1**

(12) **EUROPEAN PATENT SPECIFICATION**

(45) Date of publication of patent specification: **19.02.86**

(21) Application number: **82850084.3**

(22) Date of filing: **22.04.82**

(51) Int. Cl.⁴: **A 61 F 5/44**

(54) **Colostomy pouch.**

(30) Priority: **23.04.81 SE 8102569**

(43) Date of publication of application:
**03.11.82 Bulletin 82/44**

(45) Publication of the grant of the patent:
**19.02.86 Bulletin 86/08**

(84) Designated Contracting States:
**AT BE CH DE FR GB IT LI NL**

(56) References cited:
**DE-A-2 620 129**
**DE-A-2 928 274**
**GB-A-1 596 047**
**US-A-3 055 368**
**US-A-3 759 260**
**US-A-4 185 630**
**US-A-4 211 224**

(73) Proprietor: **SVEN HAGBERG LÄKARPRAKTIK AB**
**Järnvägsgatan 13 B**
**S-281 00 Hässleholm (SE)**

(72) Inventor: **Hagberg, Sven Johan Oskar**
**Järnvägsgatan 13 B**
**S-281 00 Hässleholm (SE)**

(74) Representative: **Lenz, Franz et al**
**AWAPATENT AB Box 5117**
**S-200 71 Malmö (SE)**

Courier Press, Leamington Spa, England.

**Description**

The present invention relates to a colostomy pouch of the type stated in the preamble of claim 1.

In colostomy pouches of this type, a small aperture is made upon application of the pouch so that gas collected in the pouch during use thereof can leak out. However, such gas leakage causes considerable discomfort to the wearer who does not know when gas leakage will occur, resulting in the spreading of offensive odours. To solve this problem, filter cartridges or capsules filled with active carbon have been developed which are intended to be placed over the gas exhausting aperture. Although the spreading of offensive odours is prevented in this manner, there is one further aspect to the application of the pouch, and this is the attachment of the filter cartridge, which may be difficult for older people. In addition, a cartridge of this type makes the colostomy pouch rather expensive.

US—A—4,211,224 relates to a colostomy pouch of the above-mentioned type, which is provided with a filter cartridge containing a fibrous filtering material which is impregnated with a deodorizing agent. The cartridge is closed at one end by means of a plug but is open or provided with an annulus of porous granular stone at the opposite end. A filtering device of this kind becomes relatively expensive because a special cartridge must be connected to the bag, thus easily giving rise to leakage in the joint between bag and cartridge. Merely using a volatile deodorizing agent and not a proper filtering material, such as active carbon, is disadvantageous and, moreover, the problem of closing the filter space at both ends has not been solved, which is necessary in order to ensure that the filter material should not deteriorate when stored. US—A—4 185 630 discloses a better solution than the first mentioned publication since the filter space is made of the bag material itself. The filter space is closed outwardly by means of a cut-off portion, but as in the former instance there is no seal between filter space and the main space of the bag.

The object of the present invention is to provide a colostomy pouch having a filter space which is sealed all along its periphery and which can be opened with respect both to the surrounding atmosphere and to the main internal space of the bag in a simple fashion. This object is achieved by means of the device defined in the characterizing clause of claim 1.

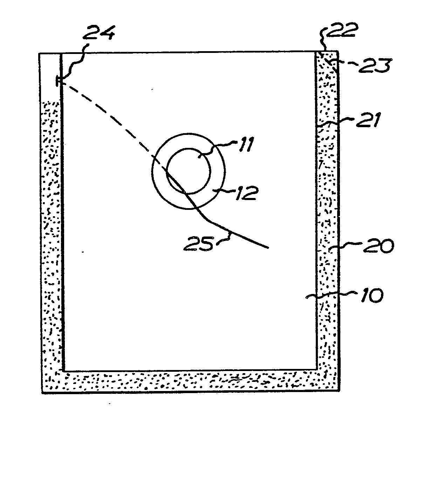

The invention will be described in more detail in the following, reference being had to the accompanying drawing illustrating an embodiment schematically and in vertical projection.

In the drawing, is presented a colostomy pouch of gas and liquid impermeable material, preferably medical quality PVC. The pouch has an inlet aperture 11, around which there is provided, in conventional manner, a sealing material 12 or like arrangment by which the pouch can be tightly sealed to an abdominal opening which, in its turn, communicates with the small or the large intestine. There are different ways in which this sealing can be effected, but since this has nothing to do with the present invention, it is merely pointed out that the pouch can be tightly sealed to the abdominal opening by means of the arrangement 12.

A partition 21 extending along three sides of the pouch and defining a main internal space (10) separated from an elongate conduit along these sides, said conduit accommodating the filter material 20 especially active carbon and gas-permeable porous material, such as cotton wool. In use, the partition is perforated to form a venting aperture at one end of the conduit, while the opposite end of said conduit is opened to the atmosphere, although initially said conduit preferably is closed both towards the interior of the pouch and to the atmosphere. The venting aperture is established by means of a cord 25 extending from the conduit through the partition, through the interior space of the pouch and outward through the inlet aperture 11 so that it can be grasped. Within the conduit, the cord end is connected with a plastic tab 24 or the like. By pulling on the cord, the plastic tab is pulled through the partition and forms the venting aperture. The pouch corner (22) is provided with an indication (23) showing how this corner should be cut off to form the gas exhausting aperture with respect to the atmosphere.

Applying the pouch according to the invention is extremely simple. Thus, the user merely has to expose the aperture 11 in conventional manner and then, by a pair of scissors or a knife, to cut off one pouch corner along the indication (23), whereupon the pouch is ready for use. Instead of removing the corner along the indication (23), it is, of course, also possible to perforate the pouch material by means of a needle or the like adjacent the corner. In any case, using the colostomy pouch according to the present invention is extremely simple since the exact perforation of the pouch material which is required in connection with filter cartridges is no longer necessary. Besides, a space provided in this manner and filled with active carbon not only eliminates offensive odours, but also provides for an advantageous equalization of pressure. In addition to active carbon and porous material, the said space may contain a deodorizing substance.

It will be readily understood that the present invention can be modified in different ways regarding both its shape and the arrangement of the separate space which is filled with filter material. The easiest way in which this space is provided has been described above, but it is also possible to dispose between the pouch sides a cord or a thin strip having one or more apertures at suitable locations, and tightly to connect the pouch sides with the opposite side faces of the cord or strip, for example by welding. In other words, the invention is not restricted to the embodiments described above and illustrated in the drawing, but can be modified in several ways within the scope of the appended claims.

## Claims

1. A colostomy pouch of a material impervious to gas and liquid and having an inlet aperture (11) and means (12) for tightly sealing said aperture to an abdominal opening, a partition (21) in the pouch extending along part of the pouch periphery and defining in the pouch a main space (10) separated from an elongate conduit along said periphery, said conduit containing a filter material (20), such as active carbon, and having at one end a gas exhausting outlet towards the atmosphere or indication for such outlet (23) in the pouch wall and an indication for a venting aperture in the partition (21) between the main space (10) and the conduit at the opposite end of said conduit, characterized in that there is provided a cord (25) extending through the partition (21), the main space (10) of the pouch and outward through the inlet aperture (11), the cord end located within the conduit being connected with a plastic tab (24) or the like adapted to be pulled through said partition by means of said cord to form said venting aperture.

2. A colostomy pouch as claimed in claim 1, characterized in that a gas-permeable porous material is provided in said conduit space, both in connection with the venting aperture and at the place of the gas exhausting outlet or indication thereof.

3. A colostomy pouch as claimed in any one of the preceding claims, characterized in that said conduit also contains a deodorizing substance.

## Revendications

1. Poche de colostomie formée d'une matière imperméable aux gaz et aux liquides et ayant une ouverture d'entrée (11) et un dispositif (12) destiné à faire coopérer de manière étanche l'ouverture avec une ouverture abdominale, une cloison (21) placée dans la poche le long d'une partie de la périphérie de la poche et délimitant dans celle-ci un espace principal (10) séparé d'un conduit allongé le long de la périphérie, le conduit contenant une matière de filtration (20) telle que du charbon actif, et ayant, à une première extrémité, une sortie d'évacuation de gaz vers l'atmosphère ou une indication pour la formation d'une telle sortie (23) dans la paroi de la poche et une indication d'une ouverture évent dans la cloison (21) entre l'espace principal (10) et l'extrémité du conduit, caractérisée en ce qu'elle comporte un cordon (25) passant dans la cloison (21), l'espace principal (10) de la poche et sortant par l'ouverture d'entrée (11), l'extrémité du cordon placée dans le conduit étant raccordée à une patte (24) de matière plastique ou analogue destinée à être tirée à travers la cloison par le cordon afin qu'elle forme l'ouverture évent.

2. Poche de colostomie selon la revendication 1, caractérisée en ce qu'une matière poreuse perméable aux gaz est placée dans l'espace du conduit, à la fois par rapport à l'ouverture évent et à l'emplacement de la sortie d'évacuation de gaz ou de l'indication d'une telle sortie.

3. Poche de colostomie selon l'une quelconque des revendications précédentes, caractérisée en ce que le conduit contient aussi une substance désodorisante.

## Patentansprüche

1. Kolostomiebeutel aus gegenüber Gas und Flüssigkeit undurchlässigem Material, mit einer Einlassöffnung (11), Mitteln (12) zum sicheren Abdichten der genannten Öffnung gegen eine Bauchöffnung, und einer Zwischenwand (21) im Beutel, die sich längs eines Teils des Beutelumfangs erstreckt und im Beutel einen Hauptraum (10) abgrenzt, der von einem langgestreckten, sich längs des genannten Umfangs erstreckenden Kanal getrennt ist, welcher ein Filtermaterial (20), wie Aktivkohle, enthält und am einen Ende eine Gasauslassöffnung zur Umgebungsluft oder eine Markierung für eine solche Auslassöffnung (23) in der Beutelwand sowie am gegenüberliegenden Ende eine Markierung für eine Entlüftungsöffnung in der Zwischenwand (21) zwischen dem Hauptraum (10) und dem Kanal aufweist, gekennzeichnet durch eine Schnur (25), die sich durch die Zwischenwand (21), den Hauptraum (10) des Beutels und durch die Einlassöffnung (11) nach aussen erstreckt, wobei das im Kanal befindliche Schnurende mit einer Kunststoffzunge (24) od.dgl. verbunden ist, die zur Bildung der Entlüftungsöffnung mittels der Schnur durch die Zwischenwand gezogen wird.

2. Kolostomiebeutel nach Anspruch 1, dadurch gekennzeichnet, dass im Kanalraum sowohl in Verbindung mit der Entlüftungsöffnung als auch an der Stelle der Gasauslassöffnung oder deren Markierung ein gasdurchlässiges, poröses Material angeordnet ist.

3. Kolostomiebeutel nach einem der vorhergehenden Ansprüche, dadurch gekennzeichnet, dass der Kanal auch desoderisierendes Mittel enthält.